# EUROPÄISCHE PATENTSCHRIFT

(11) **EP 0 566 535 B1**
(45) Veröffentlichungstag und Bekanntmachung des Hinweises auf die Patenterteilung: **11.08.1999**
(21) Anmeldenummer: 93810246.4
(22) Anmeldetag: 06.04.1993
(51) Int. Cl.: A61K 31/66, A61K 47/48

(54) **Methandiphosphonsäure-Formulierungen mit Ionenaustauschern**
Compositions comprising methanediphosphonic acids and ion-exchangers
Compositions à base d'acides méthanediphosphoniques et d'échangeurs d'ions

(30) Priorität: 15.04.1992 CH 124792
(43) Veröffentlichungstag der Anmeldung: 20.10.1993
(73) Patentinhaber: Novartis AG, 4058 Basel (CH); Novartis-Erfindungen Verwaltungsgesellschaft m.b.H., 1235 Wien (AT)
(72) Erfinder: Khanna, Satish Chandra, Dr., CH-4103 Bottmingen (CH); Green, Jonathan, Dr., CH-4144 Arlesheim (CH)

(56) Entgegenhaltungen:
- EP-A- 0 274 346
- EP-A- 0 407 344
- EP-A- 0 421 921
- GB-A- 2 118 042

## Beschreibung

Die Erfindung betrifft eine besonders vorteilhafte orale Darreichungsform für Methandiphosphonsäure-Derivate, Verfahren zur Herstellung dieser Darreichungsform sowie die Anwendung dieser Darreichungsform in einem therapeutischen Verfahren zur Behebung von Störungen des Calciumstoffwechsels.

Zahlreiche Methandiphosphonsäure-Derivate unterschiedlicher Struktur sind bekannt. So sind beispielsweise 3-Amino-1-hydroxypropan-1,1-diphosphonsäure und ihre Salze, Verfahren zur Herstellung dieser Säure sowie ihre technische Verwendung als Calciumkomplex-bildender Waschmittelbestandteil in der Deutschen Auslegeschrift Nr. 2 130 794 beschrieben worden. Die Eignung der genannten Säure und ihrer Salze als pharmazeutischer Wirkstoff ist in der Deutschen Offenlegungsschrift Nr. 2 405 254 beschrieben worden. Das Dinatriumsalz - im folgenden Dinatriumpamidronat (engl. Freiname disodium pamidronate) genannt - ist als Antihyperkalzämikum bereits klinisch untersucht worden. Zahlreiche Publikationen belegen die gute Wirksamkeit von Methandiphosphonsäure-Derivaten gegen besonders ernst zu nehmende Zustände wie Osteoporose, Osteolyse infolge Metastasen in der Knochensubstanz und Pagetsche Krankheit.

Eine antihyperkalzämisch wirksame Verbindung sollte sich ausserdem für die Langzeittherapie eignen, die bis zu mehreren Monaten oder Jahren dauern kann. Für derart lange Anwendungszeiträume sind Darreichungsformen erforderlich, welche eine Verabreichung ausserhalb des klinischen Bereichs durch den Patienten ohne fremde Hilfe ermöglichen. Perorale Darreichungsformen wie Tabletten, Dragées oder Kapseln können diesen Anforderungen entsprechen.

Vom Wirkstoff Dinatriumpamidronat ist aus *in-vivo* Befunden an Ratten die geringe Absorptionsfähigkeit von ca. 0,2 % nach oraler Verabreichung bekannt, siehe P.H.Reitsma et al. in Calcified Tissue Int.(1983) **35**: 357-361. Erforderlich wäre daher eine hohe Dosierung dieses Wirkstoffs in oralen Darreichungsformen. Diese weist aber Nachteile auf, da im British Medical J., Volume 295, 1301-1305 (1987), siehe Seite 1304, "epigastric complaints" bei klinischen Versuchen an Patienten nach Verabreichung von Kapseln oder Tabletten mit Dinatriumpamidronat erwähnt werden. Bei anderen Methandiphosphonsäure-Derivaten besteht eine ähnliche Problematik. Für orale Darreichungsformen mit niedriger Dosierung und verbesserter Magenverträglichkeit des Wirkstoffs besteht daher ein grosses Bedürfnis.

In der publizierten Europäischen Patentanmeldung Nr. 421 921 sind doppelt beschichtete Granulate, insbesondere Pellets, enthaltend den Wirkstoff Dinatriumpamidronat beschrieben, welche mit einer hydrophilen, elastischen inneren Beschichtung und einer magensaftresistenten, darmsaftlöslichen äusseren Beschichtung überzogen sind. Diese Granulate bzw. Pellets, die in Kapseln abgefüllt oral verabreichbar sind, zeichnen sich zwar durch verbesserte Magenverträglichkeit aus. Die Freisetzung erfolgt verzögert im Duodenum nach der Magenpassage, indem durch Einwirkung des Darmsaftes die magensaftresistente äussere Beschichtung langsam aufgelöst wird, so dass die Resorption des Wirkstoffes erst in diesem Bereich des Gastrointestinaltraktes erfolgt.

Wegen des Retardeffekts dieser Darreichungsform muss der Wirkstoff trotz des Risikos der Schädigung von Schleimhaut im Duodenum jedoch immer noch hoch dosiert werden (höher als ca. 150 mg Wirkstoff pro Dosiseinheitsform). Bezogen auf die Gesamtdauer des Transportvorganges durch den Gastrointestinaltrakt bewirkt nämlich der Retardeffekt eine Verkürzung der für den Resorptionsvorgang zur Verfügung stehenden Zeit. Diese zeitliche Verkürzung wird durch eine Dosiserhöhung ausgeglichen, um im verfügbaren Zeitraum, welcher für den Resorptionsvorgang noch verbleibt, eine therapeutisch wirksame Reduzierung des Calciumspiegels im Plasma zu erzielen.

Der vorliegenden Erfindung liegt die Aufgabe zugrunde, eine niedriger dosierbare Darreichungsform herzustellen, mit vorzugsweise geringer als 150 mg, insbesondere geringer als 100 mg, Wirkstoff pro Dosiseinheitsform. Bei einer derart verminderten Dosierung besteht ein geringeres Risiko der Schädigung von Schleimhäuten im gesamten Gastrointestinaltrakt und Auftreten von anderen Beschwerden wie Übelkeit.

Diese Aufgabe wird durch die vorliegende Erfindung gelöst, welche eine pharmazeutische Zusammensetzung zum Gegenstand hat und
a) ein Methandiphosphonsäure-Derivat der Formel: 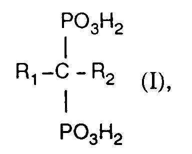 worin einer von R₁ und R₂ Wasserstoff oder Hydroxy und der andere Amino-C₁₋₄alkyl, C₂₋₆Alkylenamino-C₁₋₄alkyl, N-Mono- oder N,N-Di-C₁₋₈alkylamino-C₂₋₄alkyl, C₅₋₇Cycloalkylamino, Heteroaryl-C₁₋₄alkyl oder N-C₁₋₄alkyl-N-phenylthio-C₁₋₄alkylamino-C₁₋₄alkyl bedeuten, oder Salze davon;
b) kationisches, macroporöses Ionenaustauscherharz auf der Basis eines Styrol-Divinylbenzol-Copolymerisats mit einer austauschfähigen Aminophosphonatgruppe und gegebenenfalls
c) weitere pharmazeutisch annehmbare Hilfsstoffe enthält.

In einer besonders bevorzugten Ausführungsform enthält die pharmazeutische Zusammensetzung Dinatriumpamidronat in einer Dosierung pro Dosiseinheitsform von 50-100 mg, insbesondere 50-80 mg, im Gemisch mit dem kationischen Ionenaustauscherharz Duolite® (Warenzeichen der Fa.Rohm & Haas) C 467.

Die pharmazeutische Zusammensetzung ist vorzugsweise in Form von Tabletten, Dragées oder Kapseln anwendbar und zeichnet sich durch besonders markante Reduktion des Calciumspiegels im Plasma aus. Aus in-vivo Befunden an Ratten geht hervor, dass sich mit der neuen Darreichungsform die Absorptionsfähigkeit des Wirkstoffs von Methandiphosphonsäure-Derivaten oral verabreicht in einer öligen oder wässrigen Suspension im Gemisch mit einem Überschuss Ionenaustauscherharz bezogen auf den Wirkstoff im Gewichtsverhältnis von 1:10 um mehr als das sechsfache steigern lässt, wenn man eine oral verabreichte wässrige Suspension des Wirkstoffs zum Vergleich heranzieht. Die anhand von *in-vivo* Befunden ermittelte wirksame Dosis liegt bei ungefähr 8 mg/kg (p.o.).

Die weiter vorn und im folgenden genannten Begriffe und Definitionen haben im Rahmen der Beschreibung der Erfindung vorzugsweise die folgenden Bedeutungen:

Der Begriff pharmazeutische Zusammensetzung definiert Gemische von Methandiphosphonsäure-Derivaten (I) mit Harzpartikeln des weiter vom definierten kationischen Ionenaustauschers und gegebenenfalls üblichen pharmazeutischen Hilfsstoffen, das sich zu oralen Darreichungsformen wie Tabletten, Kapseln oder Dragées verarbeiten lässt.

Ebenfalls Gegenstand der vorliegenden Erfindung ist ein Verfahren zur Herstellung der pharmazeutischen Zusammensetzung, welches dadurch gekennzeichnet ist, dass man
a) mindestens ein Methandiphosphonsäure-Derivat (I) und
b) Harzpartikel eines kationischen Ionenaustauschers auf der Basis eines Styrol-Divinylbenzol-Copolymerisats mit einer austauschfähigen Aminophosphonatgruppe und gegebenenfalls
c) weitere pharmazeutisch annehmbare Hilfsstoffe miteinander vermischt und das Gemisch zu einer oralen Darreichungsform weiterverarbeitet.

In einer Verbindung (I) ist Amino-C₁₋₄alkyl ist vorzugsweise 2-Amino-1-ethyl oder 3-Amino-1-propyl.

C₂₋₆Alkylenamino-C₁₋₄alkyl ist vorzugsweise 2-(α,ω-C₂₋₄alkylenamino)-1-ethyl, z.B. 2-(1,4-Butylenamino)-1-ethyl.

N-Mono- oder N,N-Di-C₁₋₈alkylamino-C₂₋₄alkyl ist vorzugsweise 2-(N-C₁₋₄Alkyl-N-C₄₋₈alkylamino)-1-ethyl, z.B. 2-(N-Methyl-N-n-pentylamino)-1-ethyl.

C₅₋₇Cycloalkylamino ist vorzugsweise Cycloheptyl- oder Cyclohexylamino.

Heteroaryl-C₁₋₄alkyl ist vorzugsweise Azaaryl-C₁₋₄alkyl mit fünf oder sechs Ringgliedern, z.B. 2-, 3- oder 4-Pyridylmethyl.

N-C₁₋₄Alkyl-N-phenylthio-C₁₋₄alkylaminoC₁₋₄alkyl ist z.B. 2-[N-Methyl-N-(2-phenylthio-1-ethyl)-amino]-1-ethyl.

Salze von Methandiphosphonsäure-Derivaten sind insbesondere pharmazeutisch annehmbare Salze welche mit Aminen gebildet werden, vor allem aber Alkalimetallsalze, z.B. Natrium- oder Kaliumsalze.

Besonders bevorzugt sind die folgenden Verbindungen:

3-Amino-1-hydroxypropan-1,1-diphosphonsäure, 4-Amino-1-hydroxy-n-butan-1,1-diphosphonsäure, 3-(1,4-Butylenamino)-1-hydroxypropan-1,1-diphosphonsäure, 1-Hydroxy-3-(N-methyl-N-n-pentylamino)-propan-1,1-diphosphonsäure, 1-Cyclohexylaminomethan-1,1-diphosphonsäure, 1-Hydroxy-2-(3-pyridyl)-ethan-1,1-diphosphonsäure, 1-Hydroxy-3-[N-methyl-N-(2-phenylthio-1-ethyl)-amino]-propan-1,1-diphosphonsäure und Salze dieser Verbindungen.

Die besonders bevorzugte Komponente a) Dinatrium-3-amino-1-hydroxypropan-1,1-diphosphonat (Dinatriumpamidronat) ist in der pharmazeutischen Zusammensetzung vorzugsweise in Form des kristallinen Hydrats, vorzugsweise des Pentahydrats, vorhanden, dessen Herstellung und charakteristische Daten in der publizierten Europäischen Patentanmeldung 177 443 beschrieben sind.

Eine vorteilhafte orale Dosierung beträgt ca. 20-150 mg, insbesondere ca. 50-100 mg, vor allem ca. 50-80 mg des Wirkstoffs pro Dosiseinheitsform.

Die Komponente b) besteht aus Harzpartikeln eines kationischen, mikroporösen oder vorzugsweise makroporösen Ionenaustauschers auf der Basis eines Styrol-Divinylbenzol-Copolymerisats mit einer austauschfähigen Aminophosphonatgruppe. Die Matrix besteht aus in wässriger Phase quellfähigem, polymerisiertem Styrol mit Divinylbenzol als Vernetzungsmittel und funktionellen Aminophosphonatgruppen, welche durch austauschfähige Kationen, insbesondere Natriumionen, beladen sind. In der Handelsware Duolite® C 467 besteht die Aminophosphonatgruppe aus der Phosphonsäuremethylaminomethyl-Gruppe (-CH₂-NH-CH₂-PO₃H₂) in der Form des Kationen-austauschfähigen Mono- oder Dinatriumsalzes (-CH₂-NH-CH₂-PO₃HNa bzw. -CH₂-NH-CH₂-PO₃Na₂). Die Handelsware Duolite® C 467 hat folgende charakteristische Daten:
- Aussere Erscheinung: Beigefarbene Perlen
- Ionische Form: Na⁺
- Austauschkapazität: 1 Äquivalent pro Liter (Na⁺Form)
1,4 Äquivalent pro Liter (H⁺Form)
- Spezifisches Gewicht: 1.12 (Na⁺Form)
- Suspension: 740 g/l
- Partikelgrösse: 0.3-1 mm
- Quellvermögen: 35 % (H⁺Form ⇒ Na⁺Form)

Die Kenndaten wurden dem Product Data Sheet DTS 0092 A (publiziert im Juni 1991) der Fa. Rohm & Haas entnommen.

Die durchschnittliche Teilchengrösse der Harzpartikel beträgt ca. 1-200 µm, insbesondere 10-100 µm. Die Vernetzung beträgt ca. 2-8 %, vorzugsweise 2-4 %.

In einer besonderen Ausführungsform beträgt das Mischungsverhältnis von Methandiphosponsäure-Derivat (I) zu den Harzpartikeln des Ionenaustauschers ca. 1:1 bis 1:100, vorzugsweise ca. 1:1 bis 1:20, insbesondere 1:1 bis 1:10.

Die Komponente c) besteht aus pharmazeutisch annehmbaren Hilfsstoffen, welche für die Herstellung von oralen Darreichungsformen, z.B. festen Dosiseinheitsformen wie Tabletten, Dragées, Kapseln oder Sachets, aber auch flüssigen Darreichungsformen wie Sirupen, Tropfen, Suspensionen, Emulsionen verwendbar sind.

Tabletten erhält man durch Direktverpressen der Komponenten a) und b) mit üblichen Hilfsstoffen wie Lactose, Mannit, mikrokristalline Cellulose oder Talk oder vorzugsweise durch Verpressen von Granulaten.

Granulate sind ebenfalls feste Arzneimittelzubereitungen, welche das Methandiphosphonsäure-Derivat (I) und das weiter vom definierte Ionenaustauscherharz und solche Hilfsstoffe enthalten, die in der Pharmazeutischen Technologie für Tablettierverfahren üblich sind. Die Granulate gemäss vorliegender Erfindung sind auch selbst als orale Darreichungsformen verwendbar, indem man diese beispielsweise in Kapseln oder Sachets abfüllt. Sie werden aber vorzugsweise zu Tabletten weiterverarbeitet.

Für die Herstellung von Granulaten geeignete Hilfsstoffe sind z.B. pulverförmige Füllstoffe mit fliessregulierenden Eigenschaften, z.B. Talkum, Siliciumdioxid, z.B. synthetische amorphe entwässerte Kieselsäure vom Typ Syloid®(Grace), z.B. SYLOID® 244 FP, mikrokristalline Cellulose, z.B. vom Typ Avicel® (FMC Corp.), z.B. der Typen AVICEL® PH 101, 102, 105, RC 581 oder RC 591, Emcocel® (Mendell Corp.) oder Elcema® (Degussa), Kohlehydrate wie Zucker, Zuckeralkohole, Stärke oder Stärkederivate, z.B. Lactose, Dextrose, Saccharose, Glucose, Sorbit, Mannit, Xylitol, Kartoffel-, Mais-, Reis- oder Weizenstärke oder Amylopektin, Tricalciumphosphat, Calciumhydrogenphosphat oder Magnesiumtrisilicat, Bindemittel wie Gelatine, Traganth, Agar, Alginsäure, Celluloseäther, z.B. Methylcellulose, Carboxymethylcellulose oder Hydroxypropylmethylcellulose, Polyäthylenglycole bzw. Aethylenoxidhomopolymere, insbesondere mit einem Polymerisationsgrad von ca. 2,0 x 10³ - 1,0 x 10⁵ und einem ungefähren Molekulargewicht von ca. 1,0 x 10⁵ - 5,0 x 10⁶, z.B. unter der Bezeichnung Polyox® (Union Carbide) bekannte Hilfsstoffe, Polyvinylpyrrolidon bzw. Povidone, insbesondere mit einem mittleren Molekulargewicht von ca. 10000-360000, Polyvinylalkohol mit einem Hydrolysegrad von ca. 95-99 % und einem Polymerisationsgrad von ca. 500-2500, sowie Agar oder Gelatine, grenzflächenaktive Stoffe, z.B. anionische Tenside vom Typ Alkylsulfat, z.B. Natrium-, Kalium-oder Magnesium-n-dodecyl-sulfat, -n-tetradecyl-sulfat, -n-hexadecyl-sulfat oder -n-octadecyl-sulfat, Alkylethersulfat, z.B. Natrium-, Kalium- oder Magnesium-n-dodecyloxyethylsulfat, -n-tetradecyloxyethylsulfat, -n-hexadecyloxyethylsulfat oder -n-octadecyloxyethylsulfat oder Alkansulfonat, z.B. Natrium-, Kalium- oder Magnesium-n-dodecansulfonat, -n-tetradecansulfonat, -n-hexadecansulfonat oder -n-octadecansulfonat, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestem wie Polyoxyethylen-sorbitanmonolaurat, -oleat, -stearat, -palmitat, -tristearat oder - trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronics® (BWC) oder Synperonic® (ICI).

In einer besonderen Ausführungsform können in Tabletten auch Hilfsstoffe enthalten sein, die zur Herstellung von Brausetabletten üblich sind, nämlich jeweils mindestens ein CO₂-abgabefähiger und mindestens ein die Abgabe von CO₂ induzierender Hilfsstoff.

Ein CO₂-abgabefähiger Hilfsstoff ist z.B. ein pharmazeutisch annehmbares ein-oder zweibasisches Salz der Kohlensäure, z.B. Natrium- oder Kaliumcarbonat, insbesondere Natriumhydrogencarbonat.

Ein die Abgabe von CO₂ induzierender Hilfsstoff ist z.B. eine pharmazeutisch annehmbare Säure, welche in fester Form vorliegt und sich ohne Gasentwicklung mit dem Wirkstoff, dem Ionenaustauscherharz und anderen Hilfsstoffen zu Tabletten formulieren lässt. Eine geeignete Säure ist z.B. Weinsäure, Apfelsäure, Fumarsäure, Adipinsäure, Bernsteinsäure, Ascorbinsäure oder Maleinsäure. Bevorzugt ist Citronensäure.

Die Herstellung von Granulaten mit dem weiter vom definierten Ionenaustauscherharz erfolgt in an sich bekannter Weise unter Anwendung von Verfahren zur Herstellung von Aufbau- oder Abbaugranulaten.

Verfahren zur Bildung von Aufbaugranulaten arbeiten kontinuierlich, z.B. durch gleichzeitiges Besprühen der Granuliermasse mit Granulierlösung und Trocknung, z.B. in der Granuliertrommel, in Granulierkesseln, auf Granuliertellern, im Fliessbett, durch Sprühtrocknung oder Sprüherstarrung oder diskontinuierlich, z.B. in der Wirbelschicht, im Chargenmischer oder in der Sprühtrocknungstrommel.

Bevorzugt sind Verfahren zur Herstellung von Abbaugranulaten, welche diskontinuierlich erfolgen können, indem die Granuliermasse mit der zugeführten Granulierlösung zunächst ein feuchtes Aggregat bildet, das man anschliessend zu Granulaten mit der gewünschten Körnung zerkleinert, wobei man bekannte Extrusions- und Sphäronisationsverfahren anwendet. Als Extruder und Ausrunder sind z.B. Geräte der Firmen Wyss & Probst, Werner & Pfleiderer, HKD, Loser, Fuji, Nica, Caleva u.a. geeignet.

Die Granuliermasse besteht aus zerkleinertem, vorzugsweise gemahlenem und dem weiter vom definierten Ionenaustauscher, vorzugsweise mit einer mittleren Teilchengrösse kleiner als 400 µm (mehr als 90 %), und den weiter vom genannten Hilfsstoffen, z.B. pulverförmigen Füllstoffen wie mikrokristalliner Cellulose vom Typ AVICEL®. Besonders geeignet ist AVICEL® PH 102. Die Granuliermasse kann je nach angewendetem Verfahren vorgemischt oder durch Zumischen von APD-Na₂ zum Ionenaustauscherharz und zu einem oder mehreren vorgelegten Hilfsstoffen oder durch Zumischen der Hilfsstoffe zu vorgelegtem Wirkstoff erhalten werden.

Das Verpressen des Granulats zu Tablettenkernen kann in üblichen Tablettiermaschinen, vorzugsweise Exzenterpressen und Rundläuferpressen, insbesondere EKO-Korsch-Exzenter-Tablettiermaschinen, bei einem Arbeitsdruck von ca. 10 kN und höher vorgenommen werden.

Dragées stellt man z.B. durch Überziehen von Tablettenkernen mit einer je nach Bedarf dicken Film- oder Lackschicht unter Anwendung der bekannten Wirbelschichtverfahren, in Dragierkesseln oder gemäss Koazervierungsverfahren her.

Beispielsweise wird das Beschichtungsmittel im gewünschten Mengenverhältnis in Wasser gelöst oder suspendiert. Gegebenenfalls setzt man Hilfsstoffe wie Polyäthylenglycol zu. Diese Lösung oder Dispersion wird auf die Dragée- bzw. Tablettenkerne mit anderen Hilfsstoffen, z.B. Talk oder Siliciumdioxid, z.B. SYLOID® 244 FP, aufgesprüht, z.B. unter Verwendung bekannter Verfahren, wie Sprühumhüllung in der Wirbelschicht z.B. in den Systemen von Aeromatic, Glatt, Wurster oder Hüttlin (Kugelcoater) sowie im Kessel nach den unter den Bezeichnungen Accela Cota oder Tauchrohrverfahren bekannten Verfahren.

Kapseln sind vorzugsweise Steckkapseln aus Gelatine, insbesondere Hartgelatine, welche gegebenenfalls unter Zusatz von Glycerin oder Sorbit hergestellt werden, sich durch Einwirken von Magensaft ohne zeitliche Verzögerung auflösen und die Komponenten a) und b) freisetzen. Kapseln können die Komponenten a) und b) im Gemisch oder in Form von Granulaten enthalten. Dabei können weitere Hilfs- und Füllstoffe wie Lactose, Stärke, Gleitmittel wie Stärke oder Magnesiumstearat beigemischt sein. In weichen Kapseln können zusätzlich Flüsssigkeiten wie Lecithin, Fette, Öle, Paraffinöl oder flüssiges Polyethylenglycol enthalten sein. Geeignet sind, abhängig von der Dosierung, Steckkapseln der Grössen 0-4, vorzugsweise 0-2. Es eignet sich die Handelsware der Firmen Eli Lilly, Elanco, Capsugel oder Scherer.

In einer besonderen Ausführungsform können in den Kapseln Pellets enthalten sein, die nach den weiter vorn beschriebenen Granulierverfahren erhältlich sind, indem man die noch feuchte Granuliermasse Extrusions- oder Sphäronisationsverfahren unterwirft und dadurch regelmässig geformte, bevorzugt sphäroide, Granulate als Pellets erhält. Bevorzugt ist eine mittlere Korngrösse von ca. 0,5 bis 1,25 mm.

Sachets sind Behältnisse, z.B. Beutel aus Polyäthylen, kaschiertem Papier oder Aluminium, welche die Komponenten a) und b), z.B. Lecithin, enthalten. Nach dem Öffnen kann das Gemisch unmittelbar entnommen und z.B. im Gemisch mit Wasser oral verabreicht werden. In den genannten Kapseln und Sachets können die Komponenten a) und b) auch in Form von Granulaten oder Pellets enthalten sein.

Die weiter vorn beschriebenen festen Darreichungsformen können unterschiedlich geformt sein, z.B. rund, oval, oblong oder zylindrisch und unterschiedliche Grössen in Abhängigkeit von der darin enthaltenen Wirkstoffmenge haben. Sie können ausserdem transparent, farblos oder gefärbt und gegebenenfalls beschriftet sein, um den Produkten ein individuelles Aussehen bzw. sofortige Erkennbarkeit zu verleihen. Die Verwendung von Farbstoffen kann sowohl der Hebung des Aussehens als auch der Kennzeichnung des Präparats dienen.

Flüssige Darreichungsformen sind z.B. Sirupe, zu deren Herstellung man sich der üblichen Mischverfahren bedient wie sie in Hagers Handbuch der Pharmazeutischen Praxis, Springer Verlag, Band VII, Teil A, Seiten 640-644, oder in Remington's Pharmaceutical Sciences, Mack 1985, Seiten 1500-1503, beschrieben sind. Zunächst stellt man eine wässrige Suspension des Ionenaustauscherharzes mit dem gemahlenen Wirkstoff her und fügt zu dieser Suspension Hilfsstoffe wie die genannten Netzmittel, viskositätserhöhende Stoffe (Verdicker), Konservierungsstoffe, Antioxydanzien, Farbstoffe, Geschmackskorrigenzien (Aromastoffe), Zucker und Süssstoffe hinzu. Gegebenenfalls kann man durch Mahlen Partikel von Ionenaustauscherharzen mit geeigneter Grösse herstellen z.B. solche grösser als 1 µm und kleiner als 100 µm.

Zur Herstellung von oralen flüssigen Darreichungsformen wie Tropfen, Suspensionen, Emulsionen etc. bedient man sich der üblichen in Standardwerken wie Hagers Handbuch der Pharmazeutischen Praxis oder Remington's Pharmaceutical Sciences angegebenen Methodik.

Die pharmazeutischen Zusammensetzungen gemäss vorliegender Erfindung zeichnen sich wegen der vorteilhaft niedrigen Dosierung durch besonders gute gastrointestinale Verträglichkeit, insbesondere des Wirkstoffs Dinatriumpamidronat, aus. Die weiter vom genannten Darreichungsformen sind für die Behandlung von Krankheiten geeignet, die mit Störungen des Calciumstoffwechsels in Verbindung gebracht werden können, beispielsweise entzündlichen Prozessen in Gelenken, degenerativen Prozessen im Gelenkknorpel, Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, Neuritis, Bursitis, Periodontitis und Tendinitis, Fibrodysplasie, Osteoarthrose oder Arteriosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, Morbus Paget und die Osteodystrophia fibrosa, ebenso durch Tumore ausgelöste osteolytische Prozesse sowie Hyperkalzämie.

Folglich ist die Anwendung der pharmazeutischen, festen Darreichungsformen in einem therapeutischen oder prophylaktischen Verfahren des menschlichen oder tierischen Körpers ebenfalls Gegenstand der vorliegenden Erfindung.

Die folgenden Beispiele illustrieren die Erfindung. Dinatriumpamidronat abgekürzt: APD-Na₂.

### Beispiel 1

Rezeptur für Filmdragées. Mengenangabe pro Dosiseinheitsform.

| *Filmdragéekern* | |
|---|---|
| ADP-Na₂(Wirkstoff) | 75,0 mg |
| DUOLITE® C 467 kation. Ionenaustauscherharz | 425,0 mg |
| Mikrokristalline Cellulose AVICEL® PH 102 | 75,0 mg |
| Baumwollsamenöl hydr. CUTINA® | 15,0 mg |
| | ***590,0 mg*** |

| *Schutzlack* | |
|---|---|
| METHOCEL® Cellulose HPMC 603 | 10,0 mg |
| Talk | 9,5 mg |
| CREMOPHOR® PH 40 | 0,5 mg |
| *Filmdragée schutzlackiert* | ***610,0 mg*** |

Herstellung: Das kationische Ionenaustauscherharz wird bis zu einer durchschnittlichen Teilchengrösse von 10 µm in einer Luftstrahlmühle gemahlen. Man mischt 425 g Ionenaustauscherharz mit 75 g Wirkstoff und 75 g mikrokristalliner Cellulose zehn Minuten lang im Planetenmischer (Knedwood), setzt 15 g Baumwollsmenöl hinzu, zerkleinert durch ein 0,5 mm Sieb und mischt nochmals 5 Minuten lang. Die Mischung wird in einer Exzenterpresse EKO (Stempelgrösse 11,5 mm) verpresst. Diese Kerne werden mit dem Schutzlack mit der angegebenen Zusammensetzung in der Wirbelschicht (Strea 1) überzogen.

### Beispiel 2

Rezeptur für Kapselfüllung (flüssig). Mengenangabe pro Dosiseinheitsform.

| | |
|---|---|
| APD-Na₂(Wirkstoff) | 75,0 mg |
| DUOLITE® C 467 kation. Ionenaustauscherharz | 275,0 mg |
| Erdnussöl | 120,0 mg |
| Bienenwachs | 30,0 mg |
| *Kapselfüllung* | ***500,0 mg*** |
| Hartgelatinekapsel Grösse 0 | |

Herstellung: 275 g kation.Ionenaustauscherharz werden wie in Beispiel 1 angegeben gemahlen und mit 75 g Wirkstoff und 120 g Erdnussöl vermischt. Diese Mischung wird in einer Flüssigkeitsabfüllstation (Höflinger & Karg) in Hartgelatinekapseln der Grösse 0 abgefüllt.

### Beispiel 3

Rezeptur für Pellets. Mengenangabe pro Dosiseinheitsform.

| *Rohpellet* | |
|---|---|
| APD-Na₂(Wirkstoff) | 75,0 mg |
| DUOLITE® C 467 kation. Ionenaustauscherharz | 275,0 mg |
| Mikrokristalline Cellulose AVICEL® PH 102 | 48,0 mg |
| | ***398,0 mg*** |

| *Schutzlack* | |
|---|---|
| Celluloseether METHOCEL® HPM 603 | 5,0 mg |
| Talk | 4,75 mg |
| CREMOPHOR® PH 40 | 0,25 mg |
| *Rohpellet schutzlackiert* | ***408,0 mg*** |

Herstellung: 275 g kation.Ionenaustauscherharz werden wie in Beispiel 1 angegeben gemahlen und mit 75 g Wirkstoff und 48 g mikrokristalliner Cellulose gemischt. Die Mischung wird mit entminieralisiertem Wasser befeuchtet, extrudiert und pelletisiert. Die Pellets werden mit dem Schutzlack mit der angegebenen Zusammensetzung in der Wirbelschicht (Strea 1) überzogen.

### Beispiel 4

Rezeptur für Kapselfüllung (flüssig). Mengenangabe pro Dosiseinheitsform.

| | |
|---|---|
| 1-Hydroxy-3-(N-methyl-N-n-pentylamino)-propan-1,1-disphosphonsäure | 20,0 mg |
| DUOLITE® C 467 kation.Ionenaustauscherharz | 200,0 mg |
| SOFTISAN | 50,0 mg |
| MIGLYOL | 200,0 mg |
| *Kapselfüllung* | ***470,0 mg*** |
| Hartgelatinekapsel Grösse 0 | |

20,0 mg Wirkstoff und 200 g DUOLITE® werden in einer Luftstrahlmühle gemahlen und mit den angegebenen Hilfsstoffen vermischt. Man füllt die Paste in Hartgelatinekapseln der Grösse 0 ab.

### Beispiel 5

Rezeptur für Kapselfüllung (flüssig). Mengenangabe pro Dosiseinheitsform

| | |
|---|---|
| 1-Hydroxy-2-(3-pyridyl)-ethan-1,1-diphosphonsäure | 20,0 mg |
| DUOLITE® C 467 kation. Ionenaustauscherharz | 150,0 mg |
| Sojalecithin | 10,0 mg |
| Sesamöl | 250,0 mg |
| *Kapselfüllung* | ***430,0 mg*** |
| Hartgelatinekapsel Grösse 0 | |

10 g Sojalecithin werden in 250 g Sesamöl gelöst. 150 g DUOLITE® werden darin dispergiert und in einer Sandmühle nassgemahlen. Man setzt die berechnete Wirkstoffmenge hinzu und füllt diese Mischung in einer Abfüllstation für Flüssigkeiten (Höfliger und Karg) in Hartgelatinekapseln der Grösse 0 ab.

## Patentansprüche

1. Pharmazeutische Zusammensetzung für die orale Applikation von Methandiphosphonsäure-Derivaten enthaltend
a) ein Methandiphosphonsäure-Derivat der Formel: 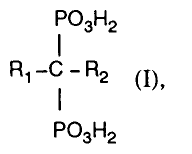 worin einer von R₁ und R₂ Wasserstoff oder Hydroxy und der andere Amino-C₁₋₄alkyl, C₂₋₆Akylenamino-C₁₋₄alkyl, N-Mono- oder N,N-Di-C₁₋₈alkylamino-C₂₋₄alkyl, C₅₋₇Cycloakylamino, Heteroaryl-C₁₋₄alkyl oder N-C₁₋₄Alkyl-N-phenylthio-C₁₋₄akylamino-C₁₋₄alkyl bedeuten, oder Salze davon;
b) kationisches, makroporöses Ionenaustauscherharz auf der Basis eines Styrol-Divinylbenzol-Copolymerisats mit einer austauschfähigen Aminophosphonatgruppe und gegebenenfalls
c) weitere pharmazeutisch annehmbare Hilfsstoffe.

2. Pharmazeutische Zusammensetzung gemäss Anspruch 1 enthaltend a) ein Methandiphosphonsäure-Derivat der Formel I, worin einer von R₁ und R₂
Wasserstoff oder Hydroxy und der andere 2-Amino-1-ethyl, 3-Amino-1-propyl, 2-(1,4-Butylenamino)-1-ethyl, 2-(N-Methyl-N-n-pentylamino)-1-ethyl, Cycloheptyl- oder Cyclohexylamino, 2-, 3- oder 4-Pyridylmethyl oder [N-Methyl-N-(2-phenylthio-1-ethyl)-amino]-1-ethyl bedeutet.

3. Pharmazeutische Zusammensetzung gemäss Anspruch 1 enthaltend
3-Amino-1-hydroxypropan-1,1-diphosphonsäure, 4-Amino-1-hydroxy-n-butan-1,1-diphosphonsäure, 3-(1,4-Butylenamino)-1-hydroxypropan-1,1-diphosphonsäure, 1-Hydroxy-3-(N-methyl-N-n-pentylamino)-propan-1,1-diphosphonsäure, 1-Cyclohexylaminomethan-1,1-diphosphonsäure, 1-Hydroxy-2-(3-pyridyl)-ethan-1,1-diphosphonsäure, 1-Hydroxy-3-[N-methyl-N-(2-phenylthio-1-ethyl)-amino]-propan-1,1-diphosphonsäure und Salze dieser Verbindungen.

4. Pharmazeutische Zusammensetzung gemäss Anspruch 1 enthaltend
a) das kristalline Pentahydrat von Dinatriumpamidronat.

5. Pharmazeutische Zusammensetzung gemäss Anspruch 4 enthaltend
a) das kristalline Pentahydrat von Dinatriumpamidronat in einer Dosierung von 50-100 mg pro Dosiseinheitsform.

6. Pharmazeutische Zusammensetzung gemäss Anspruch 5 enthaltend
a) das kristalline Pentahydrat von Dinatriumpamidronat in einer Dosierung von 50-80 mg pro Dosiseinheitsform.

7. Pharmazeutische Zusammensetzung gemäss einem der Ansprüche 1 bis 4, worin das Mischungsverhältnis der Verbindung (I) zu Harzpartikeln des Ionenaustauschers 1:1 bis 1:100 beträgt.

8. Pharmazeutische Zusammensetzung gemäss Anspruch 7, worin das Mischungsverhältnis der Verbindung (I) zu Harzpartikeln des Ionenaustauschers 1:1 bis 1:20 beträgt.

9. Pharmazeutische Zusammensetzung gemäss Anspruch 8, worin das Mischungsverhältnis der Verbindung (I) zu Harzpartikeln des Ionenaustauschers 1:1 bis 1:10 beträgt.

10. Pharmazeutische Zusammensetzung gemäss einem der Ansprüche 1-9, worin die Harzpartikel des Ionenaustauschers auf der Basis von Styrol-Divinylbenzol-Copolymerisat die austauschfähige Phosphonsäuremethylaminomethyl-Gruppe in Salzform aufweisen.

11. Pharmazeutische Zusammensetzung gemäss Anspruch 10, worin die austauschfähige Phosphonsäuremethylaminomethyl-Gruppe in der Form des Mono- oder Dinatriumsalzes vorliegt.

12. Pharmazeutische Zusammensetzung gemäss einem der Ansprüche 1-11 in Form von Tabletten, kapseln, Dragées oder Pellets.

13. Verfahren zur Herstellung einer pharmazeutischen Zusammensetzung für die orale Applikation von Methandiphosphonsäure-Derivaten, dadurch gekennzeichnet, dass man
a) eine Verbindung der Formel I gemäss Anspruch 1;
b) Harzpartikel eines kationischen Ionenaustauschers auf der Basis eines Styrol-Divinylbenzol-Copolymerisats mit einer austauschfähigen Aminophosphonatgruppe und gegebenenfalls
c) weitere pharmazeutisch annehmbare Hilfsstoffe miteinander vermischt und das Gemisch zu einer oralen Darreichungsform weiterverarbeitet.

14. Verfahren gemäss Anspruch 13, dadurch gekennzeichnet, dass man das kristalline Pentahydrat von Dinatriumpamidronat a) vermischt mit den Harzpartikeln b) und den Hilfsstoffen c) zu Tabletten, Kapseln, Dragées und Pellets verarbeitet.

15. Pharmazeutische Zusammensetzung gemäss Anspruch 1 zur Anwendung in einem therapeutischen Verfahren am menschlichen oder tierischen Körper.

16. Pharmazeutische Zusammensetzung gemäss Anspruch 15 zur Anwendung in einem therapeutischen Verfahren bei der Behebung von Störungen des Calcium-und/oder Phosphatstoffwechsels.

## Claims

1. A pharmaceutical composition for the oral administration of methanediphosphonic acid derivatives, comprising
a) a methanediphosphonic acid derivative of formula: 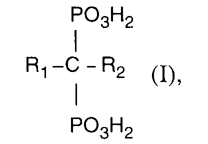 wherein one of R₁ and R₂ is hydrogen or hydroxy and the other is amino-C₁₋₄alkyl, C₂₋₆alkyleneamino-C₁₋₄alkyl, N-mono- or N,N-di-C₁₋₈alkylamino-C₂₋₄alkyl, C₅₋₇cycloalkylamino, heteroaryl-C₁₋₄alkyl or N-C₁₋₄alkyl-N-phenylthio-C₁₋₄alkylamino-C₁₋₄alkyl, or a salt thereof,
b) a cationic macroporous ion exchange resin based on a styrene/divinylbenzene copolymer having an exchangeable aminophosphonate group and, where appropriate,
c) further pharmaceutically acceptable excipients.

2. A pharmaceutical composition according to claim 1, comprising
a) a methanediphosphonic acid derivative of formula I wherein one of R₁ and R₂ is hydrogen or hydroxy and the other is 2-amino-1-ethyl, 3-amino-1-propyl, 2-(1,4-butyleneamino)-1-ethyl, 2-(N-methyl-N-n-pentylamino)-1-ethyl, cycloheptyl- or cyclohexylamino, 2-, 3- or 4-pyridylmethyl or [N-methyl-N-(2-phenylthio-1-ethyl)-amino]-1-ethyl.

3. A pharmaceutical composition according to claim 1, comprising 3-amino-1-hydroxypropane-1,1-diphosphonic acid, 4-amino-1-hydroxy-n-butane-1,1-diphosphonic acid, 3-(1,4-butyleneamino)-1-hydroxypropane-1,1-diphosphonic acid, 1-hydroxy-3-(N-methyl-N-n-pentylamino)-propane-1,1-diphosphonic acid, 1-cyclohexylaminomethane-1,1-diphosphonic acid, 1-hydroxy-2-(3-pyridyl)-ethane-1,1-diphosphonic acid, 1-hydroxy-3-[N-methyl-N-(2-phenylthio-1-ethyl)-amino]-propane-1,1-diphosphonic acid or a salt of such a compound.

4. A pharmaceutical composition according to claim 1, comprising
a) the crystalline pentahydrate of disodium pamidronate.

5. A pharmaceutical composition according to claim 4, comprising
a) the crystalline pentahydrate of disodium pamidronate in a dose of 50-100 mg per unit dose form.

6. A pharmaceutical composition according to claim 5, comprising
a) the crystalline pentahydrate of disodium pamidronate in a dose of 50-80 mg per unit dose form.

7. A pharmaceutical composition according to any one of claims 1 to 4, wherein the mixing ratio of compound (I) to the resin particles of the ion exchanger is from 1:1 to 1:100.

8. A pharmaceutical composition according to claim 7, wherein the mixing ratio of compound (I) to the resin particles of the ion exchanger is from 1:1 to 1:20.

9. A pharmaceutical composition according to claim 8, wherein the mixing ratio of compound (I) to the resin particles of the ion exchanger is from 1:1 to 1:10.

10. A pharmaceutical composition according to any one of claims 1 to 9, wherein the resin particles of the ion exchanger based on styrene/divinylbenzene copolymer comprise the exchangeable phosphonic acid methylaminomethyl group in salt form.

11. A pharmaceutical composition according to claim 10, wherein the exchangeable phosphonic acid methylaminomethyl group is in the form of the mono- or di-sodium salt.

12. A pharmaceutical composition according to any one of claims 1 to 11 in the form of tablets, capsules, dragées or pellets.

13. A process for the preparation of a pharmaceutical composition for the oral administration of a methanediphosphonic acid derivative, which comprises mixing together
a) a compound of formula I according to claim 1;
b) resin particles of a cationic ion exchanger based on a styrene/divinylbenzene copolymer having an exchangeable aminophosphonate group and, where appropriate,
c) further pharmaceutically acceptable excipients,
and further processing the mixture to produce an oral dosage form.

14. A process according to claim 13, wherein the crystalline pentahydrate of disodium pamidronate a) mixed with the resin particles b) and the excipients c) is processed to form tablets, capsules, dragées or pellets.

15. A pharmaceutical composition according to claim 1 for use in a therapeutic method for the human or animal body.

16. A pharmaceutical composition according to claim 15 for use in a therapeutic method for the alleviation of disorders of calcium and/or phosphate metabolism.

## Revendications

1. Une composition pharmaceutique pour l'administration par voie orale de dérivés de l'acide méthanediphosphonique, contenant
a) un dérivé de l'acide méthanediphosphonique de formule: 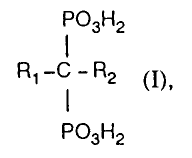 où l'un de R₁ et R₂ signifie l'hydrogène ou un groupe hydroxy et l'autre signifie un groupe amino-C₁₋₄alkyle, C₂₋₆alkylèneamino-C₁₋₄alkyle, N-mono- ou N,N-di-C₁₋₈alkylamino-C₂₋₄alkyle, C₅₋₇cycloalkylamino, hétéroaryl-C₁₋₄alkyle ou N-C₁₋₄alkyl-N-phénylthio-C₁₋₄alkylamino-C₁₋₄alkyle, ou ses sels,
b) une résine échangeuse d'ions cationique macroporeuse à base d'un copolymère de styrène/divinylbenzène ayant un groupe aminophosphonate échangeable et éventuellement,
c) d'autres excipients pharmaceutiquement acceptables.

2. Une composition pharmaceutique selon la revendication 1, contenant a) un dérivé de l'acide méthanediphosphonique de formule I, où l'un de R₁ et R₂ signifie l'hydrogène ou un groupe hydroxy et l'autre signifie un groupe 2-amino-1-éthyle, 3-amino-1-propyle, 2-(1,4-butylèneamino)-1-éthyle, 2-(N-méthyl-N-n-a) un dérivé de l'acide méthanediphosphonique de formule: ou [N-méthyl-N-(2-phénylthio-1-éthyl)-amino]-1-éthyle.

3. Une composition pharmaceutique selon la revendication 1, contenant l'acide 3-amino-1-hydroxypropane-1,1-diphosphonique, l'acide 4-amino-1-hydroxy-n-butane-1,1-diphosphonique, l'acide 3-(1,4-butylèneamino)-1-hydroxypropane-1,1-diphosphonique, l'acide 1-hydroxy-3-(N-méthyl-N-n-pentylamino)-propane-1,1-diphosphonique, l'acide 1-cyclohexylaminométhane-1,1-diphosphonique, l'acide 1-hydroxy-2-(3-pyridyl)-éthane-1,1-diphosphonique, l'acide 1-hydroxy-3-[N-méthyl-N-(2-phénylthio-1-éthyl)-amino]-propane-1,1-diphosphonique et les sels de ces composés.

4. Une composition pharmaceutique selon la revendication 1, contenant a) le pentahydrate cristallin du painidronate disodique.

5. Une composition pharmaceutique selon la revendication 4, contenant a) le pentahydrate cristallin du pamidronate disodique en une dose de 50-100 mg par dose unitaire.

6. Une composition pharmaceutique selon la revendication 5, contenant a) le pentahydrate cristallin du pamidronate disodique en une dose de 50-80 mg par dose unitaire.

7. Une composition pharmaceutique selon l'une des revendications 1 à 4, où le rapport de mélange du composé (I) aux particules de résine de l'échangeur d'ions est de 1:1 à 1:100.

8. Une composition pharmaceutique selon la revendication 7, où le rapport de mélange du composé (I) aux particules de résine de l'échangeur d'ions est de 1:1 à 1:20.

9. Une composition pharmaceutique selon la revendication 8, où le rapport de mélange du composé (I) aux particules de résine de l'échangeur d'ions est de 1:1 à 1:10.

10. Une composition pharmaceutique selon l'une des revendications 1-9, où les particules de résine de l'échangeur d'ions à base d'un copolymère de styrène/divinylbenzène, comprennent le groupe méthylaminométhyle de l'acide phosphonique échangeable, sous forme d'un sel.

11. Une composition pharmaceutique selon la revendication 10, où le groupe méthylaminométhyle de l'acide phosphonique échangeable est sous forme d'un sel mono- ou disodique.

12. Une composition pharmaceutique selon l'une des revendications 1-11, sous forme de comprimés, de gélules, de dragées ou de grains.

13. Un procédé de préparation d'une composition pharmaceutique pour l'administration par voie orale de dérivés de l'acide méthanediphosphonique, caractérisé en ce qu'on mélange ensemble
a) un composé de formule I selon la revendication 1;
b) des particules de résine d'un échangeur d'ions cationique à base d'un copolymère de styrène/divinylbenzène ayant un groupe aminophosphonate échangeable et éventuellement,
c) d'autres excipients pharmaceutiquement acceptables,
et on traite encore le mélange pour produire une formulation pour voie orale.

14. Un procédé selon la revendication 13, caractérisé en ce que le pentahydrate cristallin du pamidronate disodique a) mélangé avec les particules de résine b) et les excipients c), est mis sous forme de comprimés, de gélules, de dragées et de grains.

15. Une composition pharmaceutique selon la revendication 1, pour une utilisation dans un procédé thérapeutique pour le corps humain ou animal.

16. Une composition pharmaceutique selon la revendication 15, pour une utilisation dans un procédé thérapeutique pour soulager les troubles du métabolisme du calcium et/ou du phosphate.
